# EUROPÄISCHE PATENTSCHRIFT

(11) **EP 0 537 598 B1**
(45) Veröffentlichungstag und Bekanntmachung des Hinweises auf die Patenterteilung: **24.01.1996**
(21) Anmeldenummer: 92117050.2
(22) Anmeldetag: 06.10.1992
(51) Int. Cl.: A61B 17/56, A61B 17/60

(54) **Medizintechnisches Kompressionsimplantat**
Compressive margical implant
Implant chirurgical de compression

(30) Priorität: 18.10.1991 CH 3055/91
(43) Veröffentlichungstag der Anmeldung: 21.04.1993
(73) Patentinhaber: PINA VERTRIEBS AG, CH-8212 Neuhausen 2/SH (CH)
(72) Erfinder: Müller, Walter, W-7243 Voehringen (DE); Piotrowski, Georg, W-7238 Oberndorf (DE)
(74) Vertreter: Neymeyer, Franz, Dipl.-Ing. (FH)

(56) Entgegenhaltungen:
- EP-A- 0 383 992
- WO-A-80/01137
- WO-A-85/04096
- DE-A- 3 132 520
- DE-C- 561 561
- DE-U- 9 004 240
- US-A- 4 771 767

## Beschreibung

Die Erfindung betrifft ein medizintechnisches Kompressionsimplantat für die operative Restitution von Wirbelsäulenschäden, insbesondere im cervikalen Bereich, bestehend aus zwei gegeneinander gerichteten hakenartig gebogenen Klammerbacken, die durch eine Gewindespindel miteinander verbunden und unter Verringerung ihres Abstandes zusammenziehbar sind, wobei die Gewindespindel zwischen zwei Gewindeabschnitten mit einem Rechtsgewinde u. einem Linksgewinde ein Schlüsselprofil zum formschlüssigen Ansetzen eines Drehwerkzeuges aufweist, sowie eine Handhabungsvorrichtung für das Kompressionsimplantat.

Kompressionsimplantate der gattungsgemäßen Art werden in der chirurgischen Medizin, insbesondere bei Operationen zur Wiederherstellung der normalen Funktionen krankhafter oder verletzungsbedingter Wirbelsäulenschäden angewendet.

Wirbelsäulenverkrümmungen können beispielsweise die Folge von Wachstumsstörungen sein, welche zu keilförmigen Veränderungen der Wirbelsäule führen, oder ihre Ursache im tuberkulösen Zusammenbruch von einzelnen Wirbelkörpern haben. Es ist auch bekannt, daß Vitamin-D-Mangel zu schweren rachitischen Wirbelsäulenverkrümmungen führen kann oder durch Kalkarmut nach den Wechseljahren Wirbel in sich zusammenbrechen können. Außerdem treten immer häufiger unfallbedingte Wirbelfrakturen auf, die operativ behandelt werden müssen.

Zur Korrektur solcher Haltungsschäden oder zur Stabilisierung dieser krankheitsbedingten, respektive traumatischen Wirbelfrakturen werden heute in der Praxis einzelne Wirbel- oder Wirbelersatzstücke gegenseitig verspannt, verklammert, oder aneinander bzw. miteinander fixiert. Bisher wurden dazu metallische Drähte verwendet, um die betroffenen Wirbel zu stabilisieren bzw. die erwähnten Wirbelersatzstücke an den Wirbeln zu befestigen.

Aus der EP-A-0 383 992 ist bereits eine Vorrichtung der gattungsgemäßen Art bekannt, bei der eine Gewindespindel vorgesehen ist, die einen Gewindeabschnitt mit einem Rechtsgewinde sowie einem Gewindeabschnitt mit einem Linksgewinde u. dazwischen ein Schlüsselprofil aufweist.

Diese Gewindeabschnitte stehen jeweils im Eingriff mit entsprechenden Gewindebohrungen zweier Spannelemente, die jeweils mit einer Stützschiene verbunden sind. Die Spannelemente sind zwar um die Spindelachse der Gewindespindel drehbar u. auf dieser axial verstellbar, sie sind jedoch nicht um eine quer zur Spindelachse verlaufende Schwenkachse schwenkbar.

Es sind auch bereits schraubbare Klammern bekannt, deren Backen bei der Operation über zwei oder mehr Wirbel geschoben werden können und die mit einer herkömmlichen Schaftschraube beispielsweise wie Rohrschellen oder dergleichen zusammengezogen werden können. Diese Backen sind hakenförmig ausgebildet. Während die eine Backe eine glatte Bohrung zum Hindurchführen des Schraubenschaftes versehen ist, weist die zweite Backe eine mit einem Innengewinde versehene Bohrung auf, in welches die Schraube eingeschraubt werden kann, um die beiden Backen aufeinander zuzubewegen, wobei der Schraubenkopf am Rand der glatten Bohrung außenseitig anliegt.

Diese bekannten Klammern sind aus mehreren Gründen unbefriedigend. Vor allem ist es nicht möglich, daß eine solche Klammer von einem einzigen Operateur eingesetzt werden kann. Ihre Handhabung ist darüberhinaus sehr umständlich und insbesondere bedarf es einer großen operativen Schnittöffnung, um eine solche Klammer einsetzen zu können, da der Schraubenkopf, der am einen Ende der Schraube angeordnet ist, an dem das Drehwerkzeug beispielsweise ein abgewinkelter Schlitzschraubendreher angesetzt werden muß. Zudem müssen beide Backen, die in Verdrehung einzeln festgehalten werden, solange das Einschrauben der Schraube erfolgt, damit sie ihre funktionsgerechte Position nicht verlassen können. Aufgrund des relativ langen Spannweges, und des einfachen selbsthemmenden Gewindes sind auch sehr viele Umdrehungen der Schraube erforderlich, um die beiden Backen auf den vorgesehenen funktionsgerechten Endabstand zu bringen.

Der Erfindung liegt die Aufgabe zugrunde, ein verbessertes Kompressionsimplantat der eingangs genannten Art zu schaffen, das leichter und einfacher handhabbar und insbesondere ohne die Hilfe eines zweiten Operateurs funktionsgerecht implantiert werden kann und das trotz großer Spannweite einen nur kurzen Operationsschnitt beim Patienten erfordert.

Gelöst wird diese Aufgabe erfindungsgemäß dadurch, daß beide Klammerbacken als Gewindemuttern jeweils schwenkbar gelagerte zylindrische Drehkörper mit diametralen Gewindebohrungen zum Einschrauben der Gewindeabschnitte aufweisen.

Von besonderem Vorteil ist die an sich bekannt Verwendung einer mit zwei gegenläufigen Gewindeabschnitten versehenen Gewindespindel mit obligatorisch selbsthemmenden Gewinden, weil durch mit einer Spindelumdrehung gegenüber der bekannten Klammer die doppelte Abstandsänderung erzielt wird. Außerdem ist durch die schwenkbare Lagerung der Gewindemuttern in den beiden Klammerbacken die sehr vorteilhafte Möglichkeit gegeben, die Klammerbacken, wenn sie sich an den Enden der Gewindespindel befinden, zusätzlich auseinander zu schwenken, um eine größere Öffnungs- oder Spannweite zu erhalten.

Dadurch, daß sich das Schlüsselprofil der Gewindespindel in der Mitte zwischen den beiden gegenläufigen Gewindeabschnitten befindet, ist es auch möglich, den zur Drehung der Gewindespindel erforderlichen Schraubenschlüssel, z.B. ein Sechskantschlüssel in der Mitte des Gewindes anzusetzen. Dadurch kann die Länge des erforderlichen Operationsschnittes wesentlich kürzer gehalten werden als dies bei der Verwendung der bekannten Klammern der Fall ist. Auch kann das Auseinanderschwenken der Klammerbacken nach dem Einführen in die Operationsöffnung erfolgen. Ein weiterer Vorteil besteht darin, daß beide Klammerbacken völlig gleich ausgebildet sein können, wobei eine besonders günstige Ausgestaltung der Klammerbacken im Hinblick auf die Schwenkbarkeit und Lagerung der als Gewindemuttern ausgebildeten Drehkörpern Gegenstand des Anspruches 2 ist.

Ein sehr wesentlicher zusätzlicher Vorteil wird durch die Ausgestaltlung des Anspruches 3 insofern erzielt, als die zusätzliche Schwenkbewegung der beiden Klammerbacken um die Achse der Querbohrungen nicht nur die Verwendung einer kürzeren Gewindespindel ermöglicht, sondern auch das Erzielen einer größeren Zugbewegung der beiden Klammerbacken in der Anfangsphase des Einschraubens der Gewindespindel in die beiden Gewindemuttern. Auch dadurch wird die Handhabung wesentlich erleichtert und eine Zeitersparnis beim Einsetzen erzielt. Weitere vorteilhafte Ausgestaltungen des Kompressionsimplantates sind Gegenstand der Ansprüche 4 bis 9. Mit der Handhabungsvorrichtung der Ansprüche 10 und 11 läßt sich das erfindungsgemäße Kompressionsimplantat auf sehr vorteilhafte und insbesondere funktionsgerechte Weise sicher und positionsgenau einsetzen bzw. halten und während der Drehbewegungen der Gewindespindel gegen Verdrehen sichern, wobei gewährleistet ist, daß die beiden sich gegenüberstehenden Klammerbacken ihre Position relativ zueinander nicht verändern können. Durch die Ausgestaltung nach Anspruch 11 ist es insbesondere möglich, einen großen Schwenkbereich des am Schlüsselprofil der Gewindespindel anzusetzenden Schraubenschlüssels zu erhalten, so daß die notwendige Häufigkeit des Umsetzens des Schraubenschlüssls stark reduziert werden kann.

Anhand der Zeichnung wird ein Ausführungsbeispiel der Erfindung näher erläutert. Es zeigt:
- Fig.1: eine KLammerbacke in Draufsicht;
- Fig.2: einen Schnitt II-II aus Fig. 1;
- Fig.3: das Krallenprofil nach einem Schnitt III-III aus Fig. 2;
- Fig.4: eine Gewindespindel;
- Fig.5: die Gewindemutter in Seitenansicht;
- Fig.6: einen Schnitt VI-VI aus Fig. 5;
- Fig.7: eine andere Ausführungsform der Klammerbacke im Schnitt;
- Fig.8: das komplette Kompressionsimplantat in geschnittener Seitenansicht mit auseinandergeschwenkten Klammerbacken;
- Fig.9: das Kompressionsimplantat der Fig. 8, bei der sich die Klammerbacken in einer nur teilweise verschwenkten Lage befinden;
- Fig.10: das Kompressionsimplantat der Fig. 8 und 9, bei dem sich die beiden Klammerbacken in ihrer Normallage befinden;
- Fig.11: eine Implantierzange für das Kompressionsimplantat der Fig. 8 bis 10 in Seitenansicht;
- Fig.12: in perspektivischer Darstellung die an einem Kompressionsimplantat angesetzte Implantationszange der Fig. 11;
- Fig.13: in perspektivischer Darstellung den Endabschnitt eines fingerförmigen Greifers der Implantationszange;
- Fig.14: einen Schnitt XIV-XIV aus Fig. 13.

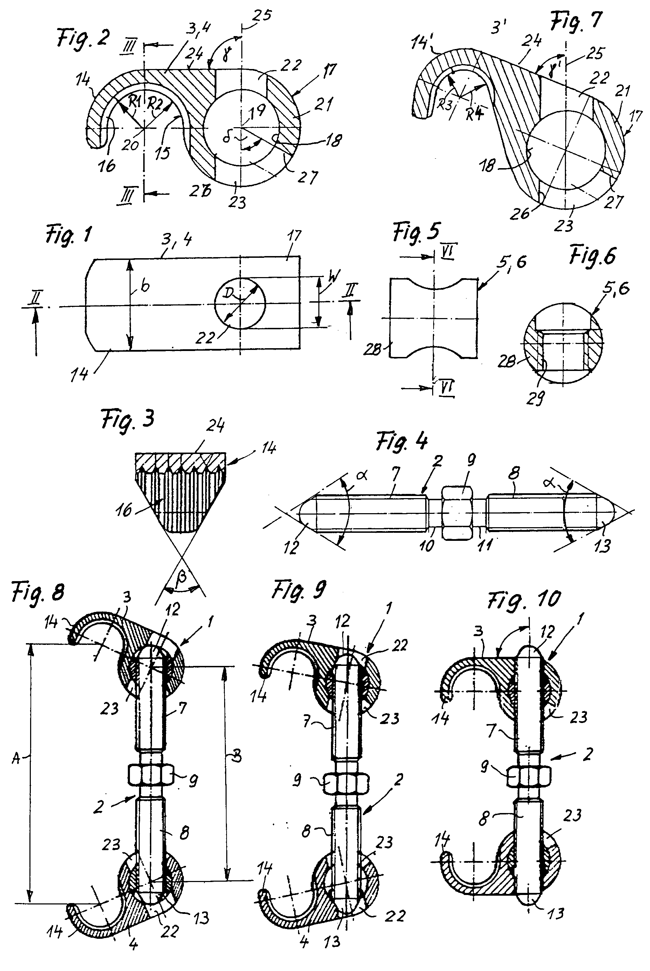

Das in den Fig. 8, 9 und 10 komplett dargestellte Kompressionsimplantat 1 besteht aus einer Gewindespindel 2 und zwei völlig gleichgestalteten Klammerbacken 3 und 4, die jeweils mit schwenkbar in ihnen gelagerten Gewindemuttern 5 und 6 versehen sind.

Die in Fig. 4 als Einzelteil dargestellte Gewindespindel 2 weist einen ersten Gewindeabschnitt 7 mit einem Rechtsgewinde und einen zweiten Gewindeabschnitt 8 mit einem Linksgewinde auf. Zwischen den beiden Gewindeabschnitten 7 und 8 befindet sich in Form eines Sechskants ein Schlüsselprofil 9, das durch zwei Einstiche 10 und 11 von den Gewinden der beiden Gewindeabschnitte 7 und 8 getrennt ist. Die stirnseitigen Enden der Gewindeabschnitte 7 und 8 sind jeweils mit einem sich nach außen hin verjüngenden, abgerundeten Konus 12 bzw. 13 versehen die jeweils einen Kegelwinkel α von etwa 60° aufweisen. Die beiden Gewindeabschnitte 7 und 8 sind jeweils mit einem metrischen Gewinde von beispielsweise 4mm Durchmesser versehen.

Die beiden gleich ausgebildeten Klammerbacken 3 und 4 sind als Einzelteil in den Fig. 1 bis 3 dargestellt. Sie weisen jeweils eine etwa halbkreisförmige Kralle 14 auf, deren Innenfläche 15 mit in Umfangsrichtung verlaufenden dreieckförmigen Rillen bzw. Zähnen 16 versehen ist und die eine insgesamt trapez- oder keilförmige Querschnittsform mit einem Keilwinkel β von etwa 60° aufweist. Die im wesentlichen halbzylindrische Innenfläche 15 der Kralle 14 weist zwei unterschiedlich große Krümmungsradien R1, R2 mit einer gemeinsamen Krümmungsachse 20 auf, wobei der größere Krümmungsradius R 2 etwa 6mm beträgt und auf der der Querbohrung benachbarten Innenseite liegt.

An die Kralle 14 schließt sich ein Rundkörper 17 an, der mit einer zylindrischen Querbohrung 18 versehen ist, deren Achse 19 parallel zur Krümmungsachse 20 der Innenfläche 15 der Kralle 14 verläuft. Die die Querbohrung 18 umschließende, teilweise zylindrische Wand 21 ist mit zwei diametralen Durchbrüchen 22 und 23 versehen, wobei der obere, d.h. auf der Rückseite 24 liegende Durchbruch 22 als Radialbohrung zur Bohrungsachse 19 und der untere Durchbruch 23 als Langloch ausgebildet sind. Die Achse 25 des als Radialbohrung ausgebildeten Durchbruches 22, welche die Achse 19 schneidet, ist gleichzeitig Krümmungsachse für den halbrunden Endabschnitt 26 des Langloches 23, während der gegenüberliegende ebenfalls halbrunde Endabschnitt 27 mit der Achse 25 einen Öffnungswinkel δ von etwa 60° bildet. Mit einer solchen Öffnungsweite läßt sich die angeschraubte Gewindespindel 2 um etwa 30° schwenken. Der Durchmesser D bzw. die Weite W der Durchbrüche 22 und 23 sind jeweils so gewählt, daß die Gewindeabschnitte 7 und 8 der Gewindespindel frei hindurchgeführt werden können. Sie beträgt im Ausführungsbeispiel etwa 4,1 mm.

Aus Fig. 2 ist ersichtlich, daß bei der dargestellten Klammerbacke 3, 4 die Achse 25 des Durchbruches 22 mit deren ebenen Rückseite 24 einen rechten Winkel γ bildet. In Fig. 7 hingegen ist eine andere Klammerbacke 3' dargestellt, bei der die Kralle 14' zwei kleinere Innenradien R3 und R4 aufweist und bei der die Achse 25 des als Radialbohrung ausgebildeten Durchbruches 22 mit der Rückseite 24 einen kleineren Winkel γ' von etwa 23° bildet. Im übrigen ist diese Klammerbacke 3' gleich ausgebildet wie die Klammerbacke 3 bzw. 4, so daß sie auch in Verbindung mit der Gewindespindel 2 und den nachfolgend noch näher beschriebenen Gewindemuttern 5 und 6 zu einem Kompressionsimplantat 1 zusammengesetzt werden kann, dessen Spannweite bei gleicher Gewindespindellänge allerdings größer ist als bei der Verwendung der Klammerbacken 3 und 4.

Die Gewindemuttern 5 und 6 sind prinzipiell gleich ausgebildet, wenn man von der Gangrichtung ihrer Innengewinde absieht. Sie bestehen, wie aus den Fig. 5 und 6 ersichtlich ist, jeweils aus einem zylindrischen Drehkörper 28 mit planparallelen Stirnflächen 26, 27 der in seiner axialen Mitte eine diametral verlaufende Gewindebohrung 29 aufweist, in welche entweder der Gewindeabschnitt 7 mit dem rechtsgängigen Gewinde oder der Gewindeabschnitt 8 mit dem linksgängigen Gewinde der Gewindespindel 2 einschraubbar ist. Demgemäß ist die Gewindebohrung 29 der Gewindemutter 5 mit einem Rechtsgewinde und der Gewindemutter 6 mit einem Linksgewinde versehen. Der Durchmesser des Drehkörpers 28 ist auf den Innendurchmesser der Querbohrung 18 so abgestimmt, daß er sich leicht in diese Querbohrung 18 einführen und drehbar in ihr lagern läßt. Seine Länge entspricht der Breite **b** der Klammerbacke 3,4.

Im zusammengesetzten Zustand ist die Gewindespindel 2 mit ihrem rechtsgängigen Gewindeabschnitt 7, der den unteren, als Langloch ausgebildeten Durchbruch 23 durchragt, in das Innengewinde 28 der Gewindemutter 5 soweit eingeschraubt, daß die Rundung des Konus 12 sich noch innerhalb der Querbohrung 18 befindet. In analoger Weise ist spiegelbildlich dazu auch der Gewindeabschnitt 8 mit dem linksgängigen Gewinde in die Gewindemutter 6 gleichweit eingeschraubt, so daß sich die beiden Klammerbacken 3 und 4 noch um die Achsen 19 ihrer Querbohrungen 18 soweit nach außen verschwenken lassen, daß die Begrenzungskanten 27 der Endabschnitte ihrer als Langlöcher ausgebildeten Durchbrüche 23 am Umfang der Gewindeabschnitte 7 bzw. 8 anliegen. Diese Position ist in Fig. 8 dargestellt. Dabei ist erkennbar, daß die Öffnungsweite **A** der beiden Greiferklammern 3 und 4 um einiges größer ist als der Abstand **B**, den in der gleichen Position die Achsen 19 der Querbohrungen 18 voneinander haben.

In diesem Zustand wird das komplettierte Kompressionsimplantat 1 operativ an den miteinander zu verbindenden Wirbeln des Patienten angesetzt, wobei diese Spreizung der Klammerbacken 3 und 4 noch nach dem Einführen in den Operationsschnitt erfolgen kann. Gegebenenfalls aber auch schon davor.

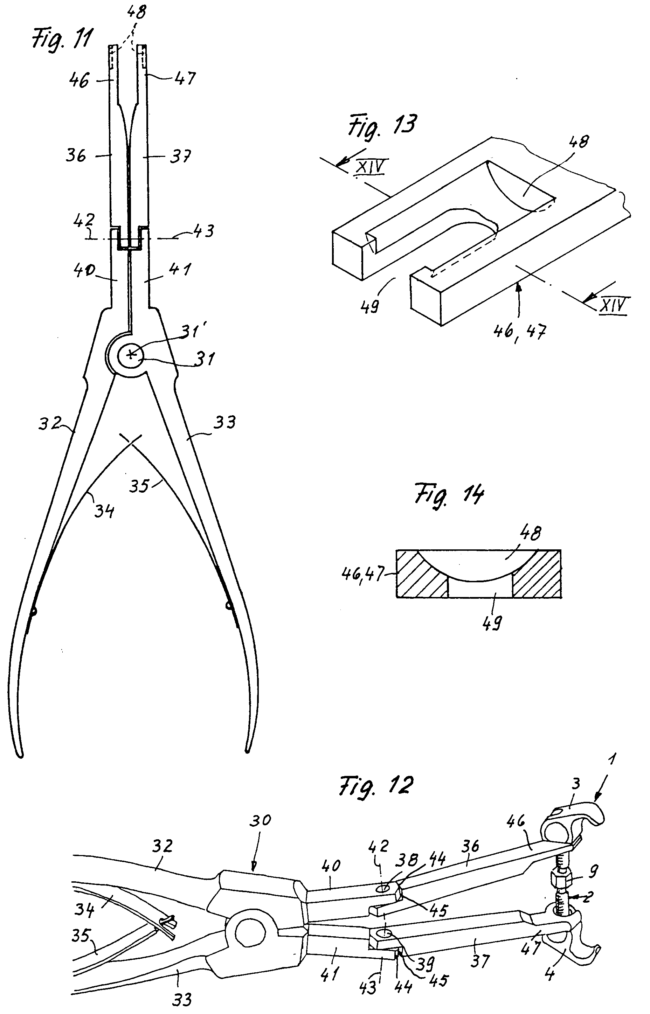

Beim späteren Drehen der Gewindespindel 2 in Spannrichtung wird die Spreizlage, wie in Fig. 8 dargestellt ist, zunehmend aufgehoben und in die Normallage der Fig. 10 übergeführt, in welcher die Achse der Gewindespindel 2 und die Achse 25 des oberen Durchbruches 22 jeweils koaxial zueinander verlaufen und auch die Gewindespindel 2 mit der Rückseite 24 der Klammerbacke 3 bzw. 4 jeweils einen rechten Winkel γ bildet.

Bewirkt wird diese Aufhebung der Spreizlage dadurch, daß die beiden Konen 12 und 13 an den Enden der beiden Gewindeabschnitte 7 und 8 in die als Radialbohrungen ausgebildeten Durchbrüche 32 eintauchen und dabei im Zusammenwirken mit den inneren Randkanten oder schrägen Leitflächen der Durchbrüche 22 ein Verschwenken der Klammerbacken 3 und 4 in Spannrichtung verursachen, so daß im Anfangsbereich des Einschraubens der Gewindespindel 2 in die beiden Gewindemuttern 5 und 6 nicht nur ein durch die Gewindegänge hervorgerufenes Zusammenführen, d.h. verkleinern des Abstandes B der beiden Klammerbacken 3 und 4 erfolgt, sondern auch jeweils eine Verschwenkbewegung in Spannrichtung hervorgerufen wird. Die Spannwirkung ist also in diesem Anfangsbereich des Spannens gemäß Fig. 8 und 9 wesentlich stärker als danach, wenn die Klammerbacken 3 und 4 ihre in Fig. 10 dargestellte Winkellage im Bezug auf die Achse der Gewindespindel 2 einnehmen.

Eine wesentliche Erleichterung der Implantation solcher Kompressionsimplantate 1 kann mit der in den Fig. 11 bis 14 dargestellten Handhabungsvorrichtung 30 erzielt werden, welche die Form einer Spreizzange aufweist. Sie besteht aus zwei durch ein Scharnier 31 gelenkig miteinander verbundenen, gebogenen Handgriffen 32 und 33, die durch zwei Blattfedern 34 und 35 auseinander gedrückt werden und die jeweils mit fingerförmigen Greifern 36 und 37 versehen sind. Diese Greifer 36 und 37 sind jeweils durch Gelenke 38 bzw. 39 mit Fingern 40 bzw. 41 der Handgriffe 32 bzw. 33 verbunden, deren Achsen 42 bzw. 43 jeweils rechtwinklig zur Scharnierachse 31' verlaufen. Durch Anschlagflächen 44 und 45 an den Fingern 40 bzw. 41 und den Greifern 36 bzw. 37 sind die Greifer 36 und 37 jeweils begrenzt aus einer mit den Fingern 40 bzw. 41 fluchtenden Mittellage nach beiden Seiten um etwa 15° schwenkbar, so daß sie in der einen Endposition etwa die in Fig. 12 dargestellte Lage einnehmen können.

Die Endabschnitte 46 und 47 der Greifer 36 und 37 sind jeweils spiegelbildlich zueinander ausgebildet und zwar jeweils mit Vertiefungen 48 versehen, welche die Querschnittsform eines Zylinderabschnitts aufweisen und in welche jeweils ein U-förmiger stirnseitig offener Schlitz 49 hineinführt. Durch diese Formgebung der Endabschnitte 46 und 47 ist es möglich, sie in der in Fig. 12 dargestellten Art an die bereits auf die Gewindespindel 2 gemäß Fig. 8 aufgeschraubten Klammerbacken 3 und 4 von innen her so anzusetzen, daß die Klammerbacken 3 und 4 formschlüssig drehfest aufgenommen werden und dabei eine zueinander parallele Lage einnehmen.
Mit Hilfe dieser Handhabungsvorrichtung 30 ist es einem Operateur leicht möglich ist, das erfindungsgemäße Kompressionsimplantat 1 ohne fremde Hilfe zu implantieren. Durch die drehfeste Aufnahme der beiden Klammerbacken 3 und 4 in den Endabschnitten 46 und 47 der Greifer 36 und 37 ist es auch leicht, die Gewindespindel 2 durch Ansetzen eines Schraubenschlüssels am Sechskant 9 in der gewünschten Richtung zu drehen, so daß eine Kompression, d.h. ein Zusammenführen der beiden Klammerbacken 3 und 4 erreicht wird.

Es ist auch denkbar, die beiden Klammerbacken 3 und 4 jeweils so auf die Gewindespindel 2 aufzuschrauben, daß ihre Krallen 14 nach außen gerichtet sind und damit ein Spreizen statt ein Zusammenziehen von Wirbeln bewerkstelligt werden kann.

## Patentansprüche

1. Medizintechnisches Kompressionsimplantat (1) für die operative Restitution von Wirbelsäulenschäden, insbesondere im cervikalen Bereich, bestehend aus zwei gegeneinander gerichteten hakenartig gebogenen Klammerbacken (3, 4), die durch eine Gewindespindel (2) miteinander verbunden und unter Verringerung ihres Abstandes (B) zusammenziehbar sind, wobei die Gewindespindel (2) zwischen zwei Gewindeabschnitten (7, 8) mit einem Rechtsgewinde und einem Linksgewinde ein Schlüsselprofil (9) zum formschlüssigen Ansetzen eines Drehwerkzeuges aufweist, **dadurch gekennzeichnet,** daß beide Klammerbacken (3 4) als Gewindemuttern (5, 6) jeweils schwenkbar gelagerte zylindrische Drehkörper mit diametralen Gewindebohrungen (29) zum Einschrauben der Gewindeabschnitte (7, 8) aufweisen.

2. Kompressionsimplantat nach Anspruch 1, dadurch gekennzeichnet, daß die Gewindemuttern (5, 6) jeweils in zylindrischen Querbohrungen (18) der Klammerbacken (3, 3) gelagert sind deren Wände (21) jeweils diametrale Durchbrüche (22, 23) zum Hindurchführen der Gewindeabschnitte (7, 8) der Gewindespindel (2) aufweisen, wobei jeweils der auf der Innenseite der Klammerbacke (3, 4) liegende Durchbruch (23) als Langloch ausgebildet ist, das einen Schwenkwinkel der hindurchragenden und in die Gewindemutter (5, 6) eingeschraubten Gewindespindel (2) um die Achse 19) der Querbohrung (18) von wenigstens 15° zuläßt.

3. Kompressionsimplantat nach Anspruch 1 oder 2, dadurch gekennzeichnet, daß der jeweils auf der Außenseite (24) der Klammerbacke liegende Durchbruch als Radialbohrung zur Achse (19) der Querbohrung ausgebildet ist und daß die Gewindespindel (2) wenigstens an einem ihrer Enden einen sich nach außen verjüngenden Konus (12, 13) aufweist, der im Zusammenwirken mit der Innenkante oder Innenfläche des als Radialbohrung ausgebildeten Durchbruchs (22) ein Verschwenken der Klammerbacke (3, 4) um die Achse 19) der Querbohrung (18) in Zugrichtung bewirkt.

4. Kompressionsimplantat nach einem der Ansprüche 1 bis 3, dadurch gekennzeichnet, daß die beiden Klammerbacken (3, 4) gleich ausgebildet sind und jeweils eine Kralle (14) aufweisen, die mit einer etwa halbzylindrischen Innenfläche (15) versehen ist, deren geometrische Krümmungsachse (20) wenigstens annähernd parallel zur Achse (19) der Querbohrung (18) verläuft.

5. Kompressionsimplantat nach Anspruch 4, dadurch gekennzeichnet, daß die Innenflächen der Krallen jeweils in Umfangsrichtung verlaufende Rillen oder Zahnprofile aufweisen.

6. Kompressionsimplantat nach Anspruch 4 oder 5, dadurch gekennzeichnet, daß die Krallen (14) an ihrer Innenfläche (15) in Umfangsrichtung verlaufende dreieckförmige Rillen oder Zähne haben, die insgesamt eine trapezförmige Querschnittsform aufweisen.

7. Kompressionsimplantat nach einem der Ansprüche 4 bis 6, dadurch gekennzeichnet, daß die Innenfläche der Kralle zwei unterschiedlich große Krümmungsradien R1, R2; R3, R4) aufweist, wobei der größere Krümmungsradius (R2 bzw. R4) auf der der Querbohrung (18) benachbarten Innenseite liegt.

8. Kompressionsimplantat nach Anspruch 2 oder 3, dadurch gekennzeichnet, daß der der Kralle (14) benachbarte, halbkreisförmige Endabschnitt (26) des als Langloch ausgebildeten Durchbruchs (23) koaxial zu dem diametral gegenüberliegenden, als Radialbohrung ausgebildeten Durchbruch (22) verläuft.

9. Kompressionsimplantat nach einem der Ansprüche 1 bis 8, dadurch gekennzeichnet, daß die Achse des als Radialbohrung ausgebildeten Durchbruchs (22) mit der Rückseite (24) der Klammerbacke (3, 4) einen rechten Winkel bildet.

## Claims

1. Compressive surgical implant (1) for operative reparation of spinal injuries, in particular in the cervical area, comprising two clamp jaws (3, 4), which are curved like hooks directed towards one another, are interconnected by a threaded spindle (2) and may be contracted to reduce the distance (B) between them, said threaded spindle (2) having a key profile (9) for form-fitting attachment of a twisting tool between two threaded sections (7, 8) with a right-hand thread and a left-hand thread, characterised in that both clamp jaws (3, 4) have respective pivotally mounted, cylindrical swivel parts as threaded nuts (5, 6) with diametric threaded holes (29) for screw attachment of the threaded sections (7, 8).

2. Compressive implant according to Claim 1, characterised in that the threaded nuts (5, 6) are each mounted in cylindrical transverse holes (18) of the clamp jaws (3, 4), the walls (21) of said transverse holes each having diametric openings (22, 23) for passage of the threaded sections (7, 8) of the threaded spindle (2), the respective opening (23) lying on the inside of the clamp jaw (3, 4) being constructed as an elongated hole, which allows the projecting threaded spindle (2) screwed into the threaded nut (5, 6) to pivot around the axis (19) of the transverse hole (18) at an angle of at least 15°.

3. Compressive implant according to Claim 1 or 2, characterised in that the respective opening lying on the outside (24) of the clamp jaw is constructed as a radial hole to the axis (19) of the transverse hole, and that at least on one of its ends, the threaded spindle (2) has a cone (12, 13) tapering to the outside, which causes the clamp jaw (3, 4) to swivel around the axis (19) of the transverse hole (18) in the direction of pull in cooperation with the inside edge or inside surface of the opening (22) constructed in the form of a radial hole.

4. Compressive implant according to one of Claims 1 to 3, characterised in that the two clamp jaws (3, 4) have the same structure and each have a claw (14), which is provided with an approximately semi-cylindrical inside surface (15), their geometric axis of curvature (20) running at least approximately parallel to the axis (19) of the transverse hole (18).

5. Compressive implant according to Claim 4, characterised in that the inside surfaces of the claws have grooves or tooth profiles running respectively in peripheral direction

6. Compressive implant according to Claim 4 or 5, characterised in that on their inside surface (15), the claws (14) have triangular grooves or teeth running in peripheral direction with an overall trapezoidal cross-sectional form.

7. Compressive implant according to one of Claims 4 to 6, characterised in that the inside surface of the claw has two radii of curvature (R1, R2; R3, R4) of different sizes, the larger radius of curvature (R2 or R4) lying on the inside adjacent to the transverse hole (18).

8. Compressive implant according to Claim 2 or 3, characterised in that the semicircular end section (26) of the opening (23) constructed in the form of an elongated hole and located adjacent to the claw (14) extends coaxially to the diametrically opposed opening (22) constructed in the form of a radial hole.

9. Compressive implant according to one of Claims 1 to 8, characterised in that the axis of the opening (22) constructed in the form of a radial hole forms a right angle with the rear side (24) of the clamp jaw (3, 4).

## Revendications

1. Implant chirurgical de compression (1) pour le rétablissement opératoire de lésions à la colonne vertébrale, dans la région cervicale en particulier, composé de deux mâchoires à crampon (3, 4) pliées en forme de crochet, dirigées l'une vers l'autre, assemblées entre elles par une tige filetée (2) et pouvant être resserrées avec une réduction de leur écartement (B), la tige filetée (2) présentant, entre deux sections filetées (7, 8) avec un filet à droite et un filet à gauche, un profil à clé (9) pour l'adaptation avec verrouillage mécanique d'un outil tournant, caractérisé en ce que les deux mâchoires à crampon (3, 4) présentent respectivement en tant qu'écrous (5, 6) des corps de rotation cylindriques logés avec une possibilité de pivotement, avec des trous taraudés diamétraux (29) pour le vissage des sections filetées (7, 8).

2. Implant de compression suivant la revendication 1, caractérisé en ce que les écrous (5, 6) sont respectivement logés dans des trous transversaux cylindriques (18) des mâchoires à crampon (3, 4), dont les parois (21) présentent respectivement des ouvertures diamétrales (22, 23) pour le passage des sections filetées (7, 8) de la tige filetée (2), l'ouverture (23) située sur le côté interne de la mâchoire à crampon (3, 4) étant réalisée sous forme de trou allongé, qui autorise un angle de pivotement de la tige filetée (2), qui la traverse et est vissée dans l'écrou (5, 6), autour de l'axe (19) du trou transversal (18) de 15° au moins.

3. Implant de compression suivant l'une des revendications 1 ou 2, caractérisé en ce que l'ouverture respectivement située sur le côté externe (24) de la mâchoire à crampon est réalisée sous forme de trou radial par rapport à l'axe (19) du trou transversal, et en ce que la tige filetée (2) présente, sur l'une de ses extrémités au moins, un cône (12, 13) rétréci vers l'extérieur qui provoque, en concours avec le rebord interne ou la surface interne de l'ouverture (22), réalisée sous forme de trou radial, un pivotement de la mâchoire à crampon (3, 4) autour de l'axe (19) du trou transversal (18), dans le sens de traction.

4. Implant de compression suivant l'une des revendications 1 à 3, caractérisé en ce que les deux mâchoires à crampon (3, 4) ont une réalisation similaire et présentent respectivement une griffe (14), munie d'une surface interne (15) à peu près semi-cylindrique, dont l'axe de courbure géométrique (20) s'étend au moins à peu près parallélement à l'axe (19) du trou transversal (18).

5. Implant de compression suivant la revendication 4, caractérisé en ce que les surfaces internes des griffes présentent respectivement des rainures ou profils dentés s'étendant dans le sens périphérique.

6. Implant de compression suivant l'une des revendications 4 ou 5, caractérisé en ce que les griffes (14) sont munies, sur leur surface interne (15), de rainures ou de dents triangulaires s'étendant dans le sens périphérique, qui présentent au total une forme trapézoïdale en coupe transversale.

7. Implant de compression suivant l'une des revendications 4 à 6, caractérisé en ce que la surface interne de la griffe présente deux rayons de courbure (R1, R2; R3, R4) de différentes valeurs, le rayon de courbure supérieur (R2 et/ou R4) se situant sur le côté interne, limitrophe au trou transversal (18).

8. Implant de compression suivant l'une des revendications 2 ou 3, caractérisé en ce que la section extrême (26) semi-circulaire, limitrophe à la griffe (14), de l'ouverture (23) réalisée sous forme de trou allongé, est coaxiale à l'ouverture (22) diamétralement opposée, réalisée sous forme de trou radial.

9. Implant de compression suivant l'une des revendications 1 à 8, caractérisé en ce que l'axe de l'ouverture (22) réalisée sous forme de trou radial forme un angle droit avec le côté arrière (24) de la mâchoire à crampon (3, 4).
